# EUROPÄISCHE PATENTSCHRIFT

(11) **EP 3 615 139 B1**
(45) Veröffentlichungstag und Bekanntmachung des Hinweises auf die Patenterteilung: **02.06.2021**
(21) Anmeldenummer: 18728011.0
(22) Anmeldetag: 26.04.2018
(51) Int. Cl.: A61N 1/39

(54) **VORRICHTUNG ZUR DEFIBRILLATION**
DEVICE FOR DEFIBRILLATION
DISPOSITIF DE DÉFRIBILLATION

(30) Priorität: 27.04.2017 DE 102017004138; 13.07.2017 DE 102017006868
(43) Veröffentlichungstag der Anmeldung: 04.03.2020
(73) Patentinhaber: WEINMANN Emergency Medical Technology GmbH + Co. KG, 22525 Hamburg (DE)
(72) Erfinder: SCHWALBE, Billy, 21129 Hamburg (DE); HERRMANN, Frank, 25355 Barmstedt (DE)
(74) Vertreter: Klickow & Wetzel PartGmbB
(86) Internationale Anmeldenummer: PCT/DE2018/000126
(87) Internationale Veröffentlichungsnummer: WO 2018/196899

(56) Entgegenhaltungen:
- DE-A1- 10 064 965
- DE-A1- 10 254 482
- DE-A1-102008 009 859
- DE-T2- 60 032 039
- US-A1- 2007 299 474

## Beschreibung

Die Erfindung betrifft eine Vorrichtung zur Defibrillation, die einen Niederspannungsbereich und einen Hochspannungsbereich aufweist und bei der der Niederspannungsbereich mit dem Hochspannungsbereich gekoppelt ist und bei der im Hochspannungsbereich Elektroden angeordnet sind.

Verfahren und Vorrichtung zur Defibrillation gehören bereits seit langem zum Stand der Technik. Beispielsweise werden entsprechende Verfahren und Vorrichtungen in der DE 1006087 B sowie der GB 864362 A beschrieben.

Gemäß dem Stand der Technik werden bei einem Defibrillator typischerweise ein oder mehrere Kondensatoren auf eine Hochspannung von etwa 2.500 V aufgeladen. Bei der Anwendung des Defibrillators an einem Patienten wird dann über einen starken Stromstoß der von den Elektroden abgegeben wird, die normale Herzaktivität wieder hergestellt. Die für den Stromstoß erforderliche Energie wird in Kondensatoren gespeichert, die im Hinblick auf die Hochspannung eine entsprechende Festigkeit aufweisen und dementsprechend teuer und schwer verfügbar sind.

Bekannt sind zwar auch bereits Defibrillatoren, bei denen sowohl ein Mehrspannungsbereich als auch ein Hochspannungsbereich verwendet wird, dieses Funktionsprinzip hat jedoch bislang keine weite Verbreitung gefunden. Ähnlich wie bei Zündspulen für Kraftfahrzeuge wird gemäß diesem Prinzip im Niederspannungsbereich ein Gleichspannungsimpuls erzeugt, der über einen Transformator geleitet wird und der aufgrund der abrupten Stromänderung im Hochspannungsbereich einen Hochspannungsimpuls erzeugt.

Gemäß den derzeit verwendeten Defibrillatoren erfolgt die Anordnung der Komponenten zur Steuerung im Hochspannungsbereich, so dass nur wenige Lieferanten zur Verfügung stehen und hohe Herstellungspreise verursacht werden.

Aus der DE 100 64 965 A1 ist bereits eine Vorrichtung zur Defibrillation bekannt, bei der ein Niederspannungsbereich durch einen Übertrager mit einem Hochspannungsbereich gekoppelt ist.

Der Hochspannungsbereich ist zur Kopplung mit Elektroden vorgesehen und der Niederspannungsbereich ist mit einem Energiespeicher ausgestattet.

In der DE 10 2008 009859 A1 wird eine weitere Vorrichtung beschrieben, die mit einem Niederspannungsbereich und einem Hochspannungsbereich versehen ist. In der US 2007/299474 A1 ist ein Defibrillator bekannt, der von einem Mikroprozessor gesteuert wird.

Eine Aufgabe der vorliegenden Erfindung ist es daher, eine Vorrichtung der einleitend genannten Art derart zu verbessern, dass mit preiswerten Komponenten eine zuverlässige Durchführung einer Defibrillation unterstützt wird.

Weitere Aufgabe der vorliegenden Erfindung ist es, eine Vorrichtung der einleitend genannten Art derart zu konstruieren, dass bei hoher Zuverlässigkeit verminderte Herstellungskosten ermöglicht werden.

Diese Aufgaben werden durch eine Vorrichtung gemäß Anspruch 1 gelöst, wodurch vermehrt elektronische Bauelemente mit einer besseren Verfügbarkeit und geringeren Kosten verwendbar sind.

Bevorzugte Ausführungsformen werden durch die abhängigen Ansprüche definiert. Aspekte, Ausführungsformen oder Beispiele in der nachstehenden Offenbarung, die nicht unter den Wortlaut der Ansprüche fallen sind nicht Teil der Erfindung. Nachstehend beschriebene Verfahren sind ebenfalls nicht Teil der Erfindung.

Durch die erfindungsgemäße Vorrichtung ist es möglich, in einem Bereich niedriger Spannung, vorteilhaft in einem Bereich der maximalen Spannung von etwa 40 V bis 400 V, besonders vorteilhaft in einem Bereich bis etwa 150 V, sowohl eine Energieversorgung als auch eine Leistungselektronik und einen Energiespeicher anzuordnen. Als Energieversorgung wird beispielsweise eine Batterie oder ein Netzteil verwendet.

Je geringer die maximale Spannung im Bereich niedriger Spannung ist, desto kostengünstiger sind in der Regel die benötigten elektronischen Bauelemente, die eine entsprechend geringere Spannungsfestigkeit aufweisen können.

Im Niederspannungsbereich einer erfindungsgemäßen Vorrichtung zur Defibrillation sind vorteilhaft zusätzlich zu dem Energiespeicher, der Leistungselektronik und der Energieversorgung, die beispielsweise als ein Netzteil oder eine Batterie bzw. ein Akku ausgebildet ist, zumindest ein Laderegler und ein Prozessor angeordnet.

Der Laderegler dient in einer bevorzugten Ausführungsform der Erfindung dem Aufladen des Energiespeichers, sodass dieser zumindest die minimal für eine Erzeugung eines Stromschocks benötigte Energie aufweist.

Der Prozessor dient in einer vorteilhaften Ausführungsform der Erfindung der Steuerung der relevanten Funktionen der erfindungsgemäßen Vorrichtung zur Defibrillation.

Der Energiespeicher gibt in einer vorteilhaften Ausführungsform der Erfindung im Moment des zu erzeugenden Schocks die Energie ab und unter Verwendung eines Spannungswandlers wird diese mithilfe eines Übertragers auf Hochspannung transformiert. Die transformierte Hochspannung kann dann über Elektroden an einen Patienten abgegeben werden.

Im Hochspannungsbereich hinter dem Übertrager (Sekundärseite) brauchen durch das erfindungsgemäße Konzept nur relativ wenige Komponenten angeordnet zu werden, Dies sind beispielsweise die für eine Phasensteuerung und eine Messvorrichtung zur Durchführung einer optionalen Strom- und/oder Spannungsmessung benötigten elektronischen Bauelemente.

Erfindungsgemäß wird insbesondere erreicht, dass es ermöglicht wird, zumindest einen Teil der Leistungselektronik und die Regeleinrichtungen für Strom, Spannung und Phase im Niederspannungsbereich anzuordnen und hierdurch preiswerte Standardkomponenten zu verwenden.

Die Durchführung der Leistungsregelung ist in einem sehr breiten Leistungsbereich möglich. Dies umfasst einen Funktionsbereich beginnend mit einer einfachen Herzschrittmacherfunktion bis zur Generierung von Hochleistungsschocks.

Insbesondere ist es möglich, den Energiespeicher in einer bevorzugten Ausführungsform der Erfindung mit preiswerten Niederspannungsbauetementen wie beispielsweise Kondensatoren und/oder Superkondensatoren und/oder (Hochleistungs-)Akkus und/oder Batterien zu realisieren.

Durch eine vorteilhaft realisierte erfindungsgemäße Energiespeicherung, ist es insbesondere auch möglich, eine sofortige Einsatzbereitschaft der Vorrichtung zur Defibrillation ohne Vorlaufzeit zur Durchführung einer Aufladung zu realisieren. Dazu ist es erforderlich, den Energiespeicher derart auszuführen, dass dieser auch in einem ausgeschalteten Zustand der Vorrichtung zur Defibrillation eine minimal zur Realisierung einer Schock- oder Herzschrittmacherfunktion benötigte Energie erhält. Darüber hinaus können mehrere Schocks in kurzen Zeitabständen relativ zueinander generiert werden.

Die eigentliche Hochspannungserzeugung erfolgt unmittelbar vor der Abgabe des Schocks. Die Systemsicherheit wird hierdurch erhöht.

Als Übertrager kann das Grundprinzip eines Transformators mit entsprechend angepassten Wicklungen verwendet werden. Für die Realisierung der Phasensteuerung sind unterschiedliche Ausführungsvarianten denkbar. Dies kann beispielsweise unter Verwendung von Dioden, Thyristoren, Triacs oder Transistoren erfolgen. Gegebenenfalls kann ein mehrstufiger Aufbau und/oder ein Aufbau mit einer Parallelschaltung von mehreren Pfaden realisiert werden.

Eine erfindungsgemäße Ausführungsform einer Vorrichtung zur Defibrillation mit einem Aufbau des Übertragers mit mehreren Sekundärwicklungen bzw. die Nutzung mehrerer in Reihe geschalteter Übertrager kann zusätzliche Vorteile generieren, da mithilfe einer geringeren Hochspannung je Wicklung eher Standardelektronik, im Sinne von kostengünstigeren elektronischen Bauelementen durch eine geringere Anforderung an die Spannungsfestigkeit, auf der Hochspannungsseite eingesetzt werden kann. Besonders vorteilhaft ist der Übertrager erfindungsgemäß auf der Primärseite mit mindestens zwei parallel geschalteten Wicklungen und auf der Sekundärseite mit mindestens zwei in Reihe geschalteten Wicklungen aufgebaut.

In einer bevorzugten Ausführungsform der Erfindung ist diese daher dadurch gekennzeichnet, dass mindestens zwei der Primär- und/oder Sekundärwicklungen der Übertrager durch eine Reihen- und/oder eine Parallelschaltung kombiniert sind.

Werden beispielsweise nur Hochspannungen von etwa 800 V bis etwa 1200 V pro Wicklung erzeugt, so kann auf eine große Palette von elektronischen Bauelementen, wie beispielsweise Halbleiterbauelemente, zurückgegriffen werden. Durch ein Kaskadieren dieser Hochspannungen lässt sich die notwendige Spannung zum Defibrillieren erzeugen. Die getrennten Wicklungen können auch dazu genutzt werden, dass beispielsweise bei der Schrittmacherfunktion nur ein Wicklungsabgriff zur Spannungserzeugung verwendet ist, da hier eine deutlich niedrigere Spannung im Vergleich zur Schockfunktion notwendig ist.

In der Erfindung ist der Spannungswandler zur Erzeugung der Hochspannung in Verbindung mit dem Übertrager als ein Resonanz-Spannungswandler realisiert, sodass sich höhere Wirkungsgrade bei der Hochspannungserzeugung erzielen lassen. Die Schaltungsverluste sind durch das Schalten bei einem Nulldurchgang der anliegenden Spannung oder einem Nulldurchgang des fließenden Stromes reduzierbar. Im Resonanzfall wird ein Stromquellenverhalten des Wandlers realisiert. Weiterhin wird eine zusätzliche Spannungsverstärkung durch die Resonanzüberhöhung erreicht.

Die Anordnung der wesentlichen Steuerungskomponenten im Niederspannungsbereich in einer vorteilhaften Ausführungsform der Erfindung führt neben der bereits erwähnten Kostenreduktion auch zu einer Unterstützung der Verkleinerung der verwendeten elektronischen Bauelemente und der benötigten Mindestabstände der spannungsführenden Bauteile für eine sichere Isolation, wodurch kompaktere Geräte realisierbar sind. Weiterhin sind durch eine erfindungsgemäße Anordnung der Komponenten einfachere und kostengünstigere Layouts der Platinen ermöglicht.

Auf der Hochspannungsseite ist zwischen dem Übertrager und dem durch die zwei Elektroden (Apex- und Sternum-Elektrode) gegeben Ausgang der Vorrichtung zur Defibrillation vorteilhaft eine Phasensteuerung bzw. sind die einer Phasensteuerung zugeordneten leistungselektronischen Komponenten angeordnet. Mit dieser Phasensteuerung lässt sich aus dem GleichspannungsAusgangssignal des Spannungswandlers, das aus dem Wechselspannungssignal auf der Sekundärseite des Übertragers mithilfe eines Gleichrichters erzeugt wird, ein biphasischer Puls generieren.

Erfindungsgemäß ist die Phasensteuerung dazu vorteilhaft als ein gesteuerter Gleichrichter ausgebildet. Beispielsweise ist der gesteuerte Gleichrichter als eine H-Brücke oder eine kaskadierte H-Brücke realisiert. Eine Ansteuerung der Transistoren erfolgt in diesem Fall vorteilhaft isoliert von der Primärseite aus. Anstelle der Dioden ist auch eine Ausführungsform mit Thyristoren angedacht, die beispielsweise über einen kleinen Hochspannungskondensator triggerbar sind. Weiterhin ist auch an eine Ausführungsform mit Triacs gedacht.

Ein beispielhaftes Verfahren zur Defibrillation umfasst zumindest die folgenden Schritte:
a. Bereitstellung der für den Stromstoß im Hochspannungsbereich benötigten Energie durch einen Energiespeicher im Niederspannungsbereich,
b. Ansteuerung eines Spannungswandlers, aufweisend mindestens einen Übertrager, durch einen im Niederspannungsbereich angeordneten Prozessor, sodass auf der Hochspannungsseite ein Hochspannungssignal bzw. ein Stromstoß generiert wird,
c. Phasensteuerung des Hochspannungssignals bzw. des Stromstoßes, sodass wahlweise ein monophasisches oder ein biphasisches Hochspannungssignal bzw. ein monophasischer oder ein biphasischer Stromstoß erzeugt wird,
d. Abgabe des Hochspannungssignals bzw. des Stromstoßes über die Elektroden an einen Patienten zur Realisierung einer Schockfunktion oder einer Schrittmacherfunktion.

Verschiedene Ausführungsbeispiele und Ausgestaltungen der Erfindung sind in den nachfolgenden Figuren abgebildet. Es zeigen
- Figur 1:: ein Blockschaltbild zur schematischen Darstellung des konstruktiven Aufbaus einer Vorrichtung zur Defibrillation,
- Figur 2:: ein detailliertes Blockschaltbild zur Veranschaulichung einer Ausführungsvariante einer Vorrichtung zur Defibrillation,
- Figur 3:: Zeitdiagramme zur Veranschaulichung des Funktionsablaufes,
- Figur 4:: eine schematische Darstellung des Aufbaus der Laderegelungsschaltung des Speicherkondensators,
- Figur 5:: eine schematische Darstellung des Schaltungsaufbaus eines Gegentaktwandlers und eines Transformators zur Übertragung der Schockenergie aus dem Niederspannungs- in den Hochspannungsbereich,
- Figur 6:: eine schematische Darstellung einer Messtelle für Strom und Spannung,
- Figur 7:: ein Schaltbild einer Ausführungsform eines mehrstufigen Übertragers,
- Figur 8:: drei schematische Blockschaltbilder zu verschiedenen erfindungsgemäßen Topologien eines Resonanzwandlers,
- Figur 9:: ein Schaltbild eines Resonanzkreises mit Übertrager und Gleichrichter und
- Figur 10:: ein Schaltbild eines Synchrongleichrichters am Beispiel eines Übertragers mit Resonanzkreis.

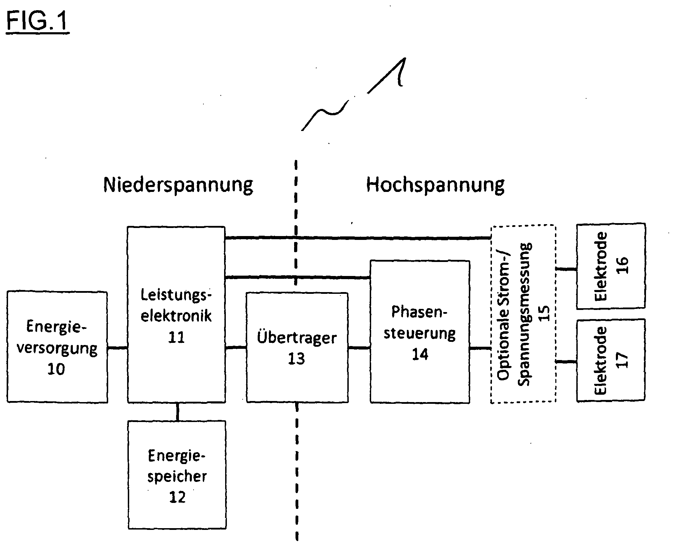

Figur 1 zeigt ein Blockschaltbild einer Vorrichtung zur Defibrillation (1) und veranschaulicht insbesondere die Kopplung des Niederspannungsbereichs mit dem Hochspannungsbereich durch einen Übertrager (13). Im Niederspannungsbereich sind eine Energieversorgung (10) und ein Energiespeicher (12) zusammen mit der Leistungselektronik (11) angeordnet.

Im Hochspannungsbereich sind die Phasensteuerung (14) und zwei Elektroden (16, 17) angeordnet. Optional kann eine Einrichtung zur Strom- und/oder Spannungsmessung (15) im Hochspannungsbereich angeordnet sein.

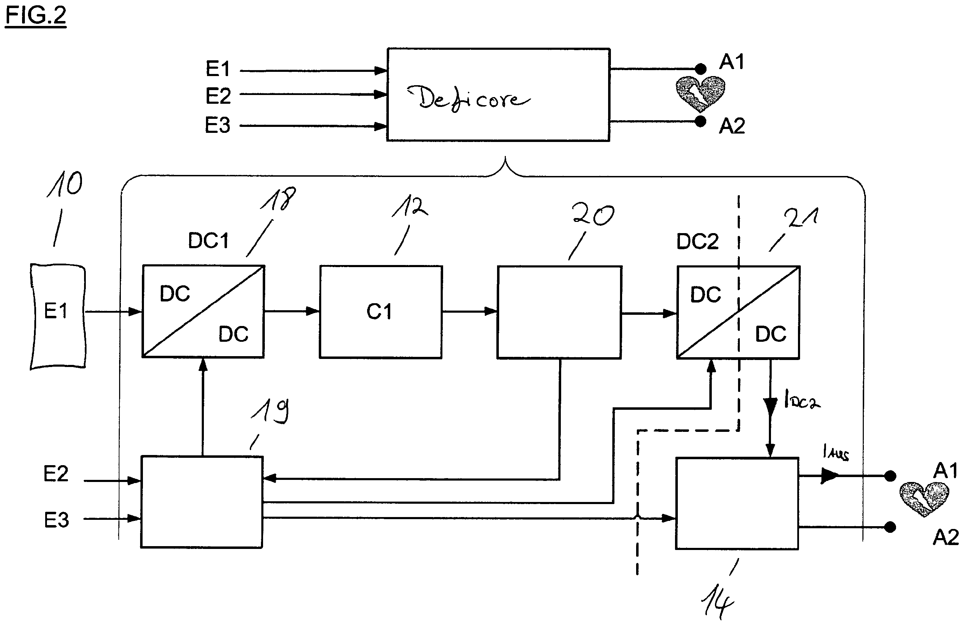

Figur 2 veranschaulicht den Aufbau des Gesamtsystems einer Ausführungsform einer Vorrichtung zur Defibrillation (1) in einer Übersichtsdarstellung. Es wird insbesondere derjenige elektronische Teil der Vorrichtung zur Defibrillation (1) veranschaulicht, mit der der Defibrillationsimpuls erzeugbar ist.

Die Eingangssignale E1, E2 und E3 dieses sogenannten Deficores, sind gegeben durch die Energieversorgung (E1), die in einer vorteilhaften Ausführungsform der Erfindung eine Spannung von etwa 12V bei einer verfügbaren Leistung von etwa 50 W bis 100 W aufweist, das Signal zur Aktivierung eines Schocks (E2) und ein Konfigurationssignal (E3), das beispielsweise über eine I2C-Schnittstelle übertragbar ist.

Mit dem Deficore ist der Defibrillationspuls über die Elektroden des Patientenanschlusses an einen Patienten abgebbar. Der Patientenanschluss weist die Anschlüsse Apex (A1) und Sternum (A2) auf.

Mit einer Vorrichtung zur Defibrillation (1) ist dazu in einer vorteilhaften Ausführungsform mithilfe des Energiespeichers (12) eine Energie von etwa 200 Ws abgebbar und ein Hochspannungsimpuls von etwa 2.000 V erzeugbar. Die mithilfe des Energiespeichers (12) speicherbare Energie setzt sich zusammen aus zumindest der abgebbaren Energie und derjenigen Energie, die benötigt wird, um die auftretenden Verluste innerhalb der Vorrichtung zur Defibrillation zu kompensieren.

Insbesondere ist daran gedacht, einen biphasischen Schock mit einer Länge von etwa 10 ms zu generieren. Die Stärke des Schockstromes beträgt typisch bis zu 20 Ampere.

Gemäß einer Ausführungsform der Vorrichtung zur Defibrillation (1) kann ein Anwender wählen, ob der Schockstrom biphasisch oder monophasisch generiert werden soll und welche Schocklänge zu erzeugen ist.

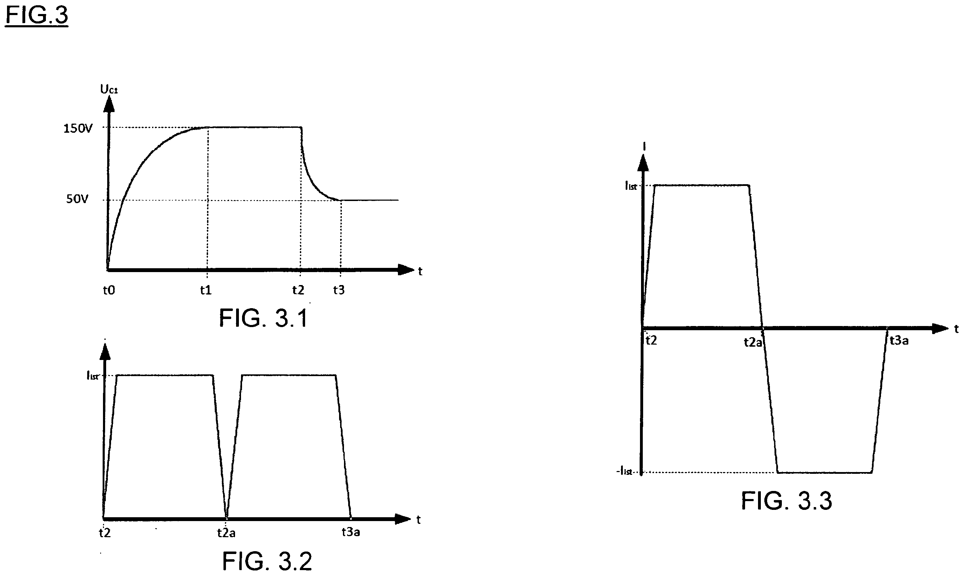

Gemäß den Darstellungen in Figur 3 wird die Funktion der Erzeugung eines biphasischen Schocks anhand eines Beispiels nochmals näher erläutert,

Figur 3.1 zeigt den Spannungsverlauf UC1 am Kondensator C1 in Abhängigkeit der Zeit t, Figur 3.2 zeigt den Stromverlauf I_{DC2} am Ausgang des Spannungswandlers DC2 (21) in Abhängigkeit der Zeit t und Figur 3.3 zeigt den Stromverlauf I_{AUS} am Ausgang der Vorrichtung zur Defibrillation (1) in Abhängigkeit der Zeit t.

Bei einem Funktionsbeginn zum Zeitpunkt t0 sind zunächst alle Spannungen auf 0 V und alle Ströme auf 0 Ampere. Beim Einschalten der Versorgungsspannung der Vorrichtung zur Defibrillation (1) erfolgt ein Start des Ladereglers (18) und dieser beginnt den als Kondensator C1 ausgebildeten Energiespeicher (12) mit dem eingeregelten Strom zu laden,

Zum Zeitpunkt t1 ist der Energiespeicher (12) voll aufgeladen und das Spannungsniveau am Kondensator C1 beträgt etwa 150 V. Das benötigte Spannungsniveau am Energiespeicher (12) hängt von der abzugebenden Energie ab. Die Ladung des Energiespeichers (12) erfolgt adaptiv und typisch auf eine Spannung bis zu etwa 200 V.

In einem weiteren Schritt löst dann der in Figur 2 dargestellte Prozessor (19) den Schock zum Zeitpunkt t2 aus und am Spannungswandler DC2 (21) wird eine Pulsweiten-Modulation angelegt. Zusätzlich wird dann die als eine H-Brücke ausgebildete Phasensteuerung (14) freigeschaltet.

In einem weiteren Verfahrensschritt regelt der Prozessor (19) durch Modulation der Pulsweiten und/oder der Frequenz und/oder der Phasenlage der Steuersignale für die Leistungselektronik den Strom des Schocks. Über den Spannungswandler DC2 (21) wird mithilfe des Übertragers (13) hierzu die Kondensatorspannung auf das benötigte Potential von bis zu etwa 2.500 V auf der Hochspannungsseite transformiert. Am Ausgang von DC2 und am Ausgang der H-Brücke stellen sich ein Strom I_{DC2} und I_{AUS} mit einer Stromstärke entsprechend I_{Ist} ein.

In einem nachfolgenden Schritt wird zum Zeitpunkt t2a der Strom I_{DC2} am Ausgang von DC2 kurzzeitig auf 0 Ampere abgesenkt und hierbei die H-Brücke umgeschaltet, um einen biphasischen Puls zu generieren.

Anschließend steigen die Ströme I_{DC2} und I_{AUS} wieder auf die Stromstärke list an, bevor sie zum Zeitpunkt t3a wieder auf 0 Ampere abgesenkt werden und die Erzeugung eines Schockpulses abgeschlossen ist.

Während der Erzeugung des Schocks mit dem Stromverlauf I_{AUS} entlädt sich der Energiespeicher (12) und die Spannung am Kondensator C1 fällt entsprechend Figur 3.1 zwischen den Zeitpunkten t2 und t3 auf eine Restspannung von etwa 50V ab. Die Restspannung ist von der benötigten Schockenergie und von der Dimensionierung des Übertragers (13) abhängig.

Gemäß einem weiteren Verfahrensschritt werden die Stromwerte und Spannungswerte, die für die Regelung erforderlich sind, vor dem Spannungswandler DC2 (21) gemessen. Generell kann auch eine Messung hinter dem Spannungswandler DC2 (21) erfolgen. Im zweiten Fall erfolgt jedoch eine Potentialtrennung zwischen dem Prozessor (19) und der Messstelle (20).

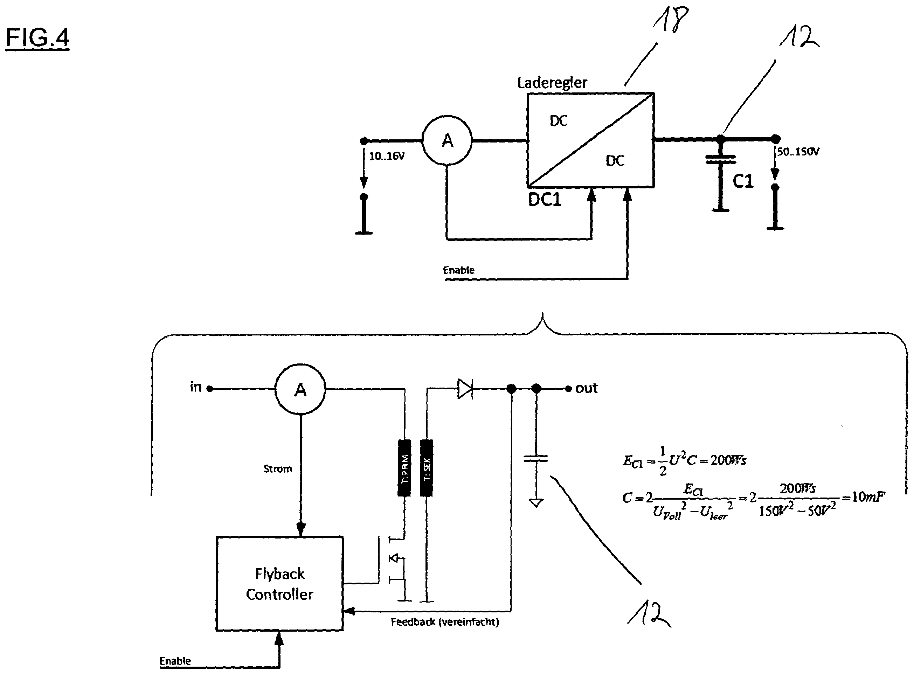

Gemäß der Darstellung in Figur 4 werden Details des Ladereglers (18) und des als Kondensator C1 ausgebildeten Energiespeichers (12) veranschaulicht.

Im Bereich des Kondensators C1 wird die für den Schock benötigte Energie zuzüglich der zur Kompensation der Verluste benötigten Energie gespeichert und der als ein Gleichstrom-Gleichstrom-Wandler realisierte Laderegler (18) lädt den Kondensator C1 auf. Bei der Aufladung des Kondensators C1 wird der Eingangsstrom vorzugsweise begrenzt.

Vorzugsweise wird zur Realisierung des Ladereglers (18) eine Flyback-Topologie gewählt, um eine hohe Spannungsdifferenz zwischen Eingang und Ausgang zu erzeugen.

Als Kondensator C1 kann beispielsweise ein handelsüblicher Elektrolytkondensator verwendet werden, es sind aber auch andere Kondensator-Typen wie beispielsweise Superkondensatoren oder keramische Kondensatoren denkbar.

Bei einer beispielhaft zu speichernden Energie von 200 Ws im Kondensator C1, wird, bei einer als etwa 150 V festgelegten anliegenden Spannung im vollständig geladenen Zustand und einer als etwa 50 V festgelegten anliegenden Spannung nach der Entladung durch die Energieabgabe bei der Erzeugung eines Schocks, ein Kondensator C1 mit einer Kapazität von etwa 10 mF benötigt. Die entsprechenden Formeln sind ohne Berücksichtigung der auftretenden Verluste in Figur 4 dargestellt.

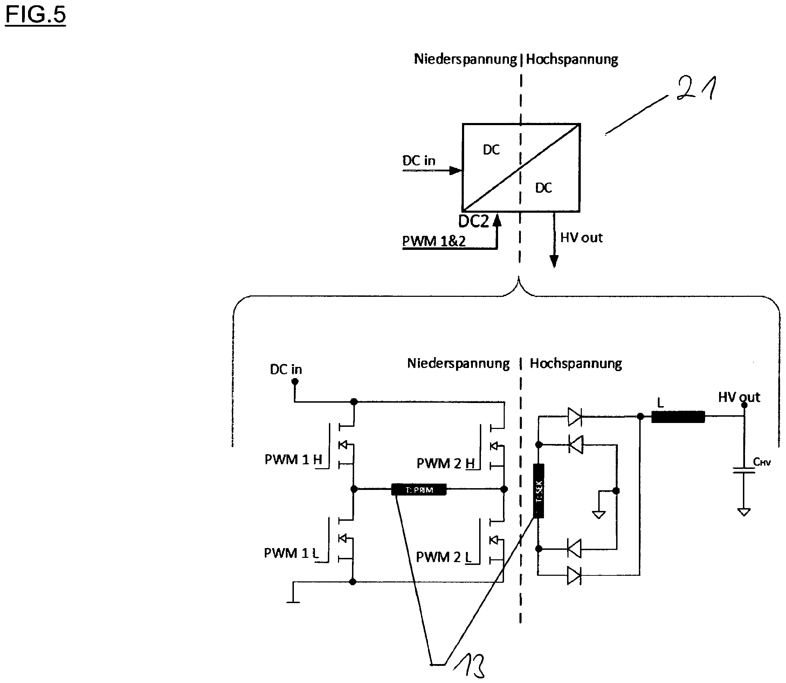

Gemäß der Darstellung in Figur 5 werden weitere Details des Spannungswandlers DC2 (21) und des Übertragers (13) veranschaulicht.

Der Spannungswandler DC2 (21) überträgt mithilfe des Übertragers (13) die Schockenergie von der Niederspannungsseite auf die Hochspannungsseite. Der Spannungswandler DC2 (21) kann beispielsweise als ein Gegentaktwandler realisiert sein.

Auf der Niederspannungsseite des Spannungswandlers DC2 (21) ist eine Vollbrückenschaltung mit Leistungstransistoren realisiert. Die Leistungstransistoren werden jeweils über ein pulsweitenmoduliertes Signal (PWM 1 H/L, PWM 2 H/L) angesteuert, sodass die Richtung des Stromflusses durch die mindestens eine primärseitige Wicklung des mindestens einen Übertragers (13) regelbar ist.

Auf der Sekundärseite des Übertragers (13) ist der Wicklung ein mit Dioden realisierter Brückengleichrichter nachgeschaltet, an den sich ein L-C-Filter anschließt. An der Klemme HV out ist somit ein Gleichspannungssignal im Hochspannungsbereich abgreifbar.

Eine Leistungsabgabe beträgt beispielsweise 20 kW für einen Zeitraum von 10 ms um eine Energie von 200 Ws abzugeben.

Der Spannungswandler DC2 (21) kann in einer bevorzugten Ausführungsform direkt durch den Prozessor (19) bzw. durch den Prozessor (19) in Verbindung mit Gatetreibern gesteuert werden.

Der als ein Transformator ausgebildete Übertrager (13) kann beispielsweise in einer Planartechnik realisiert werden. Darüber hinaus ist es auch denkbar, mehrere Transformatoren relativ zueinander parallel zu schalten und hierdurch den Spannungswandler DC2 (21) als einen multiparallelen Gegentaktwandler zu realisieren. Auch ist die Verwendung von Transformatoren mit mehreren Sekundärwicklungen und/oder die Verwendung von mehreren Transformatoren mit einer oder mehreren Sekundärwicklungen in einer Ausführungsform einer Vorrichtung zur Defibrillation (1) denkbar.

Gemäß der in Figur 5 dargestellten Ausführungsvariante wird ein typischer Hochspannungsbereich von etwa 2.000 V bis 2.500 V realisiert. Auch hier wird die Hochspannung vorzugsweise ausschließlich während der Schockabgabe erzeugt. Bei einer Energieabgabe von typisch 200 Ws und einem Patientenwiderstand von 25 bis 175 Ohm (typisch 50 Ohm), ist eine Spannung von 750 V bis 2 kV erforderlich.

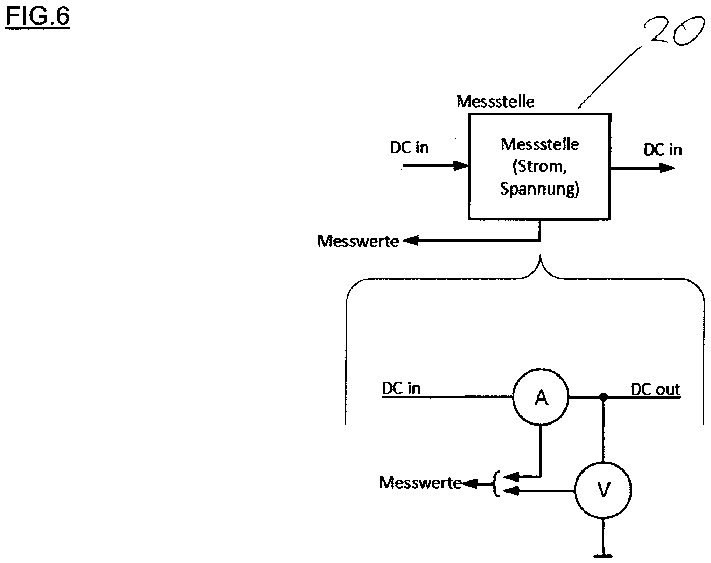

Gemäß der Darstellung in Figur 6 werden weitere Details der Strom- und Spannungsmessung veranschaulicht.

Der Entnahmestrom aus den Kondensatoren kann gemessen und der entsprechende Wert der Regelung bereitgestellt werden.

Die Messung kann kontinuierlich oder abgetastet erfolgen. Eine typische Abtastfrequenz liegt in einem Bereich von 20 bis 40 kHz. Dies entspricht etwa 200 bis 400 Messungen je Schockdauer.

Gemäß einer bevorzugten Ausführungsvariante erfolgt die Messung von Strom und Spannung hinter dem Spannungswandler DC2 (21) und vor der H-Brücke auf der Hochspannungsseite.

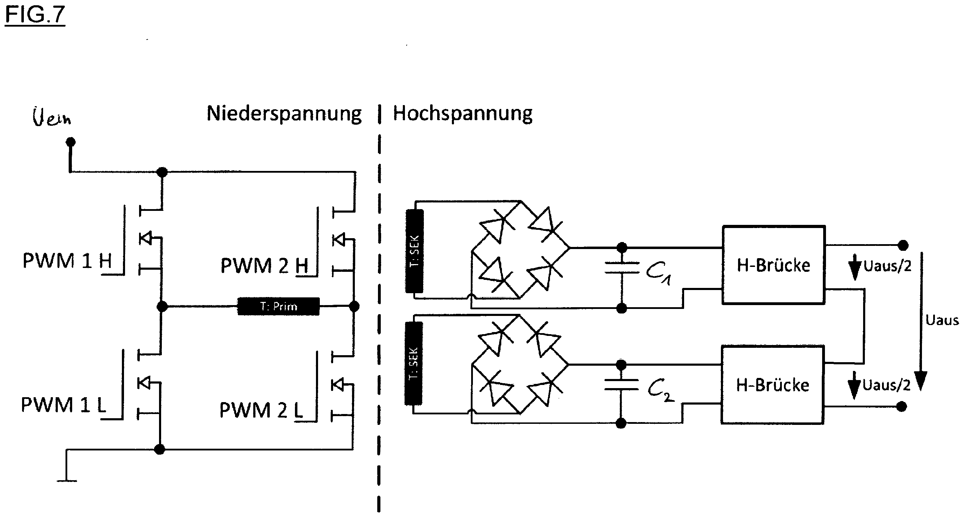

In Figur 7 ist in einer schematischen Darstellung ein Schaltbild eines beispielhaften mehrstufigen Aufbaus eines Übertragers (13) mit anschließender Gleichrichterschaltung dargestellt. Die primärseitig anliegende Spannung Uein (Eingangsspannung der H-Brücke des Inverters) ist über den Übertrager (13) in einen Hochspannungsbereich transformierbar und mithilfe von Gleichrichtern in die Ausgangsspannung Uaus wandelbar. Auf der Primärseite weist der Übertrager (13) eine Wicklung (T: Prim) auf, während auf der Sekundarseite zwei in Reihe geschaltete Wicklungen (T: SEK) angeordnet sind. Der Übertrager (13) hat beispielhaft ein Wicklungsverhältnis von 1:8.

Jeder der sekundärseitigen Wicklungen (T: SEK) des Übertragers (13) ist jeweils ein mithilfe von Dioden realisierter Brückengleichrichter und ein Kondensator (C1, C2) sowie eine H-Brücke nachgeschaltet. Über jedem der in Reihe geschalteten sekundärseitigen Schaltungszweige fällt somit nur die halbe Ausgangsspannung Uaus ab. Die Halbleiter und Kondensatoren auf der Sekundärseite benötigen somit nur noch die halbe Spannungsfestigkeit.

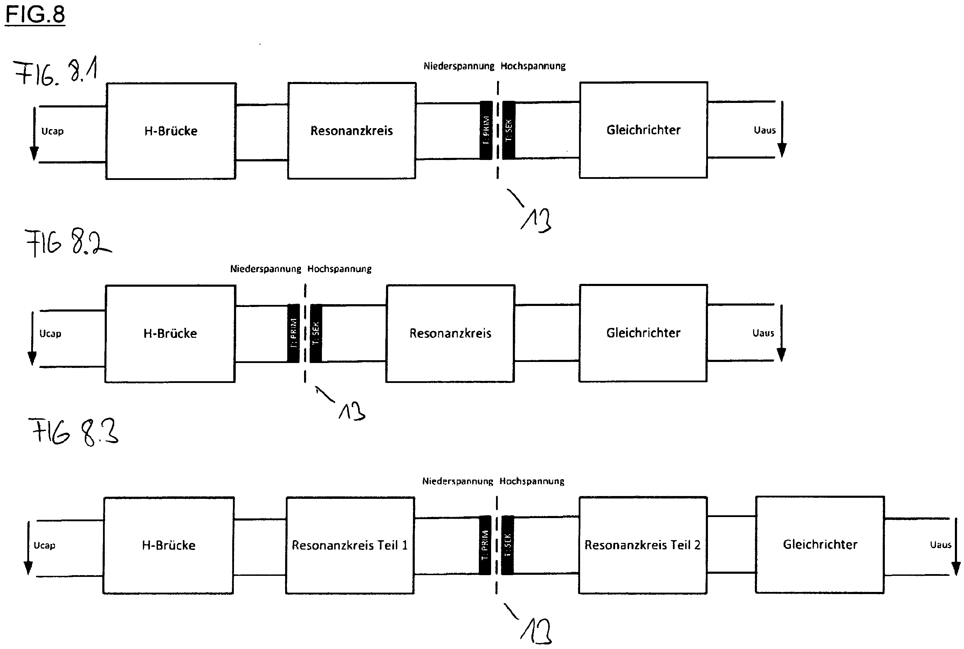

Figur 8 zeigt drei erfindungsgemäße Topologien eines Resonanzwandlers zur Transformation der Schockenergie aus dem Niederspannungs- in den Hochspannungsbereich.

Figur 8.1 zeigt eine Anordnung des Resonanzkreises zwischen der H-Brücke des Inverters und der Primärseite des Übertragers (13) im Niederspannungsbereich.

Figur 8.2 zeigt eine Anordnung des Resonanzkreises auf der Sekundärseite des Übertragers (13) vor dem Gleichrichter.

Figur 8.3 zeigt eine kombinierte Anordnung des Resonanzkreises auf Primär- und Sekundärseite des Übertragers (13).

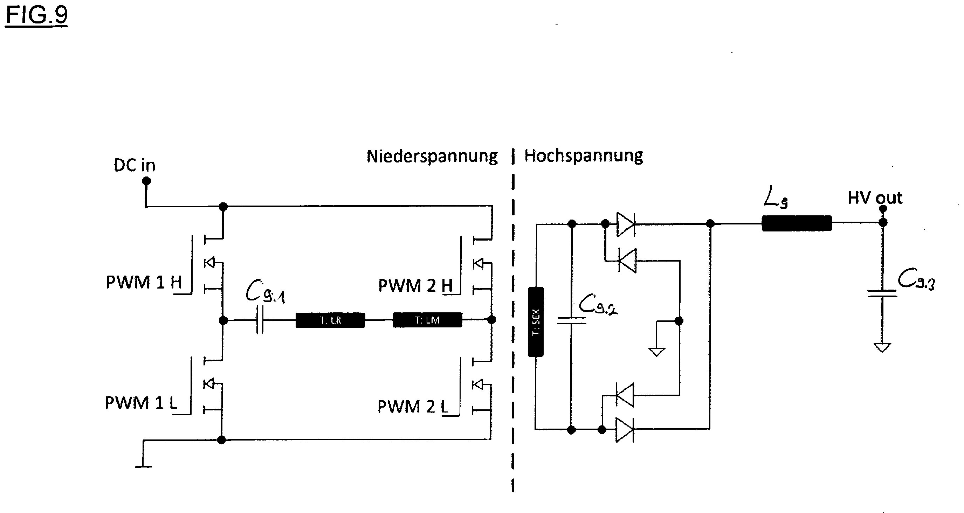

In Figur 9 ist ein Resonanzkreis mit Transformator in einem Schaltbild dargestellt. Der Resonanzkreis ist als eine kombinierte Topologie auf Primär- und Sekundärseite des Übertrages (13) realisiert.

Die Spannung DCin (Eingangsspannung des Inverters im Niederspannungsbereich) ist mithilfe der dargestellten Komponenten in die Ausgangsspannung HVout wandelbar.

Die für die Einstellung des gewünschten Arbeitspunktes (Resonanzfrequenz) des Resonanzkreises bzw. des Resonanzwandlers relevanten Komponenten sind die Streuinduktivität T:LR des Transformators bzw. Übertragers (13) und der sekundärseitige Kondensator C_{9.2} (Resonanzkondensator). Die Werte der Hauptmagnetisierungsinduktivität T:LM des Übertragers (13) und des Kondensators C_{9.1}, der zur Gleichstromunterdrückung im Übertrager (13) dient, werden in einer vorteilhaften Ausführungsform der Vorrichtung so dimensioniert, dass der gewünschte Arbeitspunkt des Resonanzkreises nicht verändert wird. Dies wird in der Regel dadurch gelöst, dass eine sich zusätzlich ergebende Resonanzfrequenz möglichst weit entfernt von der Frequenz des Arbeitspunktes platziert wird.

Dem mithilfe von Dioden realisierten Gleichrichter ist ein Filter nachgeschaltet, der durch die Komponenten Lg und C_{9.3} gegeben ist.

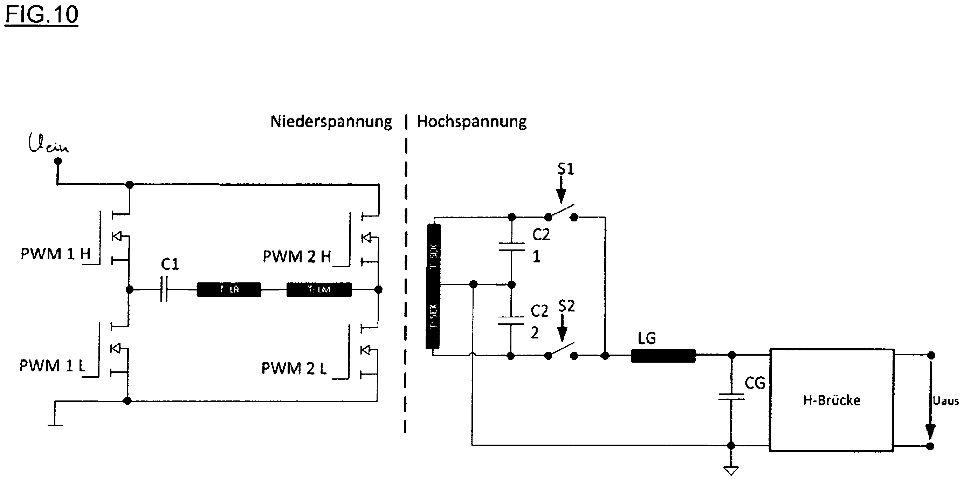

Figur 10 zeigt ein Schaltbild eines umschaltbaren Gleichrichters, ausgebildet als ein Synchrongleichrichter, am Beispiel eines Übertragers (13) mit Resonanzkreis.

Die Eingangsspannung Uₑᵢₙ ist mithilfe der dargestellten Komponenten in die Ausgangsspannung Uₐᵤₛ wandelbar.

Der sekundärseitige Gleichrichter weist die Schalter S1 und S2 auf, wobei durch eine aktive und synchrone Ansteuerung der Schalter S1 und S2 die Gleichrichterfunktion realisierbar ist.

Je nach Schaltreihenfolge von S1 und S2, ist eine positive oder negative Spannung Uₐᵤₛ erzeugbar. Die Schalter S1 und S2 müssen in ihrer Ausführungsform als Halbleiter bidirektional zu schließen und zu öffnen sein.

## Patentansprüche

1. Vorrichtung zur Defibrillation (1), die einen Niederspannungsbereich und einen Hochspannungsbereich aufweist und bei der der Niederspannungsbereich über mindestens einen Übertrager (13) mit dem Hochspannungsbereich gekoppelt ist und bei der im Hochspannungsbereich Elektroden (16, 17) angeordnet sind, wobei im Niederspannungsbereich sowohl eine Energieversorgung (10) als auch eine Leistungselektronik (11) und ein Energiespeicher (12) angeordnet sind, **dadurch gekennzeichnet, dass** der Übertrager (13) Teil eines Spannungswandlers (21) zur Wandlung des Stromstoßes aus dem Niederspannungsin den Hochspannungsbereich ist, wobei der Spannungswandler als ein Resonanz-Wandler ausgebildet ist.

2. Vorrichtung nach Anspruch 1, **dadurch gekennzeichnet, dass** eine Hochspannung temporär und bei Bedarf erzeugbar ist.

3. Vorrichtung nach mindestens einem der vorstehenden Ansprüche, **dadurch gekennzeichnet, dass** die Niederspannungsseite für einen Spannungsbereich bis zu 400 V ausgelegt ist.

4. Vorrichtung nach mindestens einem der vorstehenden Ansprüche, **dadurch gekennzeichnet, dass** diese durch einen auf der Niederspannungsseite angeordneten Prozessor (19) steuerbar ist.

5. Vorrichtung nach mindestens einem der vorstehenden Ansprüche, **dadurch gekennzeichnet, dass** der Energiespeicher (12) als ein Kondensator und/oder als ein Superkondensator und/oder als ein Akkumulator und/oder als eine Batterie ausgebildet ist.

6. Vorrichtung nach mindestens einem der vorstehenden Ansprüche, **dadurch gekennzeichnet, dass** eine Energie dauerhaft mit dem Energiespeicher (12) speicherbar ist, sodass durch die Vorrichtung (1) instantan nach der Einschaltung ein Schock erzeugbar ist.

7. Vorrichtung nach mindestens einem der vorstehenden Ansprüche, **dadurch gekennzeichnet, dass** der mindestens eine Übertrager (13) eine oder mehrere Primär- und/oder Sekundärwicklungen aufweist.

8. Vorrichtung nach Anspruch 7, **dadurch gekennzeichnet, dass** mindestens zwei der Primär- und/oder Sekundärwicklungen der Übertrager (13) durch eine Reihen- und/oder eine Parallelschaltung kombiniert sind.

9. Vorrichtung nach mindestens einem der vorstehenden Ansprüche, **dadurch gekennzeichnet, dass** die Sekundärwicklungen eines oder mehrerer Übertrager (13) derart seriell verschaltet sind, dass auf der Hochspannungsseite elektronische Bauelemente mit einer in einem Bereich von etwa 500 V bis etwa 1500 V spezifizierten maximalen Spannungsfestigkeit verwendbar sind.

10. Vorrichtung nach mindestens einem der vorstehenden Ansprüche, **dadurch gekennzeichnet, dass** sowohl ein mono- als ein biphasischer Ausgangspuls erzeugbar ist.

11. Vorrichtung nach mindestens einem der vorstehenden Ansprüche, **dadurch gekennzeichnet, dass** eine Strom- und/oder Spannungsmessung (20) auf der Primär- oder Sekundärseite des Übertragers (13) realisiert ist.

## Claims

1. Device for defibrillation (1), which has a low-voltage range and a high-voltage range and in which the low-voltage range is coupled to the high-voltage range via at least one translator (13) and in which electrodes (16, 17) are arranged in the high-voltage range, wherein both a power supply (10) and a set of power electronics (11) and an energy store (12) are arranged in the low-voltage range, **characterized in that** the translator (13) is part of a voltage converter (21) for converting the current surge from the low-voltage range into the high-voltage range, wherein the voltage converter is implemented as a resonance converter.

2. Device according to Claim 1, **characterized in that** a high voltage can be generated temporarily and on demand.

3. Device according to at least one of the preceding claims, **characterized in that** the low-voltage side is designed for a voltage range of up to 400 V.

4. Device according to at least one of the preceding claims, **characterized in that** said device can be controlled by a processor (19) arranged on the low-voltage side.

5. Device according to at least one of the preceding claims, **characterized in that** the energy store (12) is implemented as a capacitor and/or as a super-capacitor and/or as an accumulator and/or as a battery.

6. Device according to at least one of the preceding claims, **characterized in that** energy can be permanently stored with the energy store (12), so that the device (1) can generate a shock instantaneously after being switched on.

7. Device according to at least one of the preceding claims, **characterized in that** the at least one translator (13) has one or more primary and/or secondary windings.

8. Device according to Claim 7, **characterized in that** at least two of the primary and/or secondary windings of the translators (13) are combined by a series and/or a parallel circuit.

9. Device according to at least one of the preceding claims, **characterized in that** the secondary windings of one or more translators (13) are interconnected in series in such a way that electronic components with a maximum voltage breakdown strength specified in a range from approximately 500 V to approximately 1500 V can be used on the high-voltage side.

10. Device according to at least one of the preceding claims, **characterized in that** both a monophasic and a biphasic output pulse can be generated.

11. Device according to at least one of the preceding claims, **characterized in that** a current and/or voltage measurement (20) is implemented on the primary or secondary side of the translator (13).

## Revendications

1. Dispositif de défibrillation (1) qui présente une zone basse tension et une zone haute tension, et dans lequel la zone basse tension est couplée à la zone haute tension par au moins un transformateur (13), et dans lequel des électrodes (16, 17) sont disposées dans la zone haute tension, à la fois une alimentation en énergie (10) et une électronique de puissance (11) et un dispositif de stockage d'énergie (12) étant disposés dans la zone basse tension, **caractérisé en ce que** le transformateur (13) fait partie d'un convertisseur de tension (21) permettant de convertir la pointe de surtension de la zone basse tension à la zone haute tension, le convertisseur de tension étant réalisé sous la forme d'un convertisseur à résonance.

2. Dispositif selon la revendication 1, **caractérisé en ce qu'**une haute tension peut être produite temporairement et en cas de besoin.

3. Dispositif selon au moins l'une des revendications précédentes, **caractérisé en ce que** le côté basse tension est conçu pour une plage de tension jusqu'à 400 V.

4. Dispositif selon au moins l'une des revendications précédentes, **caractérisé en ce qu'**il peut être commandé par un processeur (19) disposé du côté basse tension.

5. Dispositif selon au moins l'une des revendications précédentes, **caractérisé en ce que** le dispositif de stockage d'énergie (12) est réalisé sous la forme d'un condensateur et/ou d'un supercondensateur et/ou d'un accumulateur et/ou d'une batterie.

6. Dispositif selon au moins l'une des revendications précédentes, **caractérisé en ce qu'**une énergie peut être stockée de façon permanente par le dispositif de stockage d'énergie (12) de sorte que le dispositif (1) peut produire un choc instantanément après la mise sous tension.

7. Dispositif selon au moins l'une des revendications précédentes, **caractérisé en ce que** ledit au moins un transformateur (13) présente un ou plusieurs enroulements primaires et/ou secondaires.

8. Dispositif selon la revendication 7, **caractérisé en ce qu'**au moins deux des enroulements primaires et/ou secondaires des transformateurs (13) sont combinés par une connexion en série et/ou en parallèle.

9. Dispositif selon au moins l'une des revendications précédentes, **caractérisé en ce que** les enroulements secondaires d'un ou de plusieurs transformateurs (13) sont connectés en série de telle sorte que du côté haute tension, des composants électroniques ayant une résistance au claquage maximale spécifiée comprise dans une plage d'environ 500 V à environ 1500 V peuvent être utilisés.

10. Dispositif selon au moins l'une des revendications précédentes, **caractérisé en ce qu'**à la fois une impulsion de sortie monophasée et une impulsion de sortie biphasée peuvent être produites.

11. Dispositif selon au moins l'une des revendications précédentes, **caractérisé en ce qu'**une mesure de courant et/ou de tension (20) est réalisée du côté primaire ou du côté secondaire du transformateur (13).
